# EUROPEAN PATENT APPLICATION

(11) **EP 2 987 520 A1**
(43) Date of publication of application: **24.02.2016**
(21) Application number: 14002867.1
(22) Date of filing: 18.08.2014
(51) Int. Cl.: A61M 11/00, A61M 15/00

(54) **Fluid reservoir for an aerosol generator and aerosol generator comprising the fluid reservoir**

(71) Applicant: PARI Pharma GmbH, 82319 Starnberg (DE)
(72) Inventor: Gallem, Thomas, 81371 München (DE); Hetzer, Uwe, 80687 München (DE); Knoch, Martin, 82335 Berg (DE); Neuner, Michael, 81539 München (DE)
(74) Representative: Hoffmann Eitle

(57) **Abstract**

The invention relates to a fluid reservoir (2) for an aerosol generator (50), the fluid reservoir (2) comprising a fluid chamber (4) for receiving a fluid (6), an opening (8) for guiding the fluid (6) received in the fluid chamber (4) outside the fluid chamber (4), and a collar portion (10) surrounding the opening (8) and extending into the fluid chamber (4). A first portion (12) of the fluid chamber (4) extends along the length of the collar portion (10) in a height direction (H) of the fluid chamber (4). A second portion (14) of the fluid chamber (4) is arranged adjacent to the first portion (12) in the height direction (H) of the fluid chamber (4). A lateral extension of the first portion (12) in the directions perpendicular to the height direction (H) of the fluid chamber (4) is smaller than a lateral extension of the second portion (14) in the directions perpendicular to the height direction (H) of the fluid chamber (4). Further, the invention relates to a fluid reservoir (2') for an aerosol generator (50), the fluid reservoir (2') comprising a fluid chamber (4') for receiving a fluid (6'), an opening (8') for guiding the fluid (6') received in the fluid chamber (4') outside the fluid chamber (4'), a recess (9) arranged at least partly below the opening (8') in a height direction (H) of the fluid chamber (4'), and a cover member (30') which releasably or permanently seals the recess (9). Moreover, the invention relates to an aerosol generator (50) comprising the fluid reservoir (2, 2').

## Description

### Field of the Invention

The invention relates to a fluid reservoir for an aerosol generator, the fluid reservoir comprising a fluid chamber for receiving a fluid and an opening for guiding the fluid received in the fluid chamber outside the fluid chamber. Further, the invention relates to an aerosol generator comprising the fluid reservoir.

### Background Art

Aerosols for therapeutic purposes are generated and delivered to a desired location within a user's or patient's body with aerosol delivery devices. A fluid or liquid (i.e., medicament) to be aerosolised or nebulised is received in a fluid reservoir, supplied from the fluid reservoir to an aerosol generator of the aerosol delivery device and aerosolised or nebulised by the aerosol generator. The resultant aerosol is supplied to the user or patient.

The requirements placed on the aerosol delivery device arise from the treatment to be performed with the aerosols. One of these requirements concerns dosage accuracy and precision, i.e., the accuracy and precision of the administered quantity of the medicament provided as an aerosol. Only if the dose of a medicament administered to the user or patient is precisely established, a precise and efficient treatment with a highly effective medicament can be carried out. Basic conditions influencing dosage accuracy and precision are the quantity of fluid or liquid to be aerosolised that is guided from the fluid reservoir to the aerosol generator and the aerosol generation efficiency of the aerosol generator.

The aerosol generation efficiency varies depending on the type of aerosol generator used. For example, vibrating membrane aerosol generators, e.g., vibrating membrane nebulisers, are generally more efficient in terms of aerosol generation than compressor driven jet nebulisers. This is because vibrating membrane nebulisers are able to aerosolise essentially all of the liquid filled into the reservoir, whereas jet nebulisers have an inherent residual volume left inside the nebuliser due to droplet impaction and wetting of large internal surface areas from which the recirculating liquid cannot be fully recovered. Hence, in order to match and ensure a consistent and precise aerosolised drug dosage from a given amount of fluid or liquid to be filled into the fluid reservoir of different types of aerosol generators, the aerosol generation efficiency of some types of aerosol generators, such as vibrating membrane aerosol generators, has to be artificially reduced.

Such an artificial efficiency reduction may be achieved by depositing a portion of the generated aerosol in the aerosol generator, e.g., by droplet impaction on the walls thereof, by increasing aerosol losses from the aerosol generator during the patient exhalation phase or by using a particular type of fluid or liquid reservoir, i.e., a retention reservoir, which retains a portion of the fluid or liquid in the reservoir. This retained portion of the fluid or liquid is not supplied to the aerosol generator and thus not aerosolised or nebulised. The portion remains in the fluid or liquid reservoir and is discarded after the aerosol therapy.

The use of such a retention reservoir offers the advantage of shorter inhalation times, i.e., shorter therapy times, since only a portion of the fluid or liquid received in the reservoir is aerosolised or nebulised, while achieving a specified aerosolised drug dosage.

However, known retention reservoirs are very sensitive to the angle of orientation or alignment of the reservoir, i.e., to the angle at which the aerosol generator is held, for example, by a user or patient during aerosol therapy or inhalation therapy. Depending on this angle, the portion of fluid or liquid retained in the reservoir may vary, i.e., increase or decrease, leading to undesired variations in aerosol dosage and thus compromising the aerosol therapy.

Hence, there remains a need for a fluid reservoir which allows for the amount of fluid or liquid retained in the reservoir to be kept constant over a range of alignment or orientation angles of the reservoir in each tilting or inclination direction thereof.

### Summary of the Invention

One object of the invention is to provide a fluid reservoir for an aerosol generator which ensures that the amount of fluid or liquid retained in the reservoir is kept substantially constant over a range of alignment or orientation angles of the reservoir in each tilting or inclination direction. Further, the invention aims to provide an aerosol generator comprising this fluid reservoir. These goals are achieved by a fluid reservoir with the technical features of claim 1, a fluid reservoir with the technical features of claim 12 and an aerosol generator with the technical features of claim 14. Preferred embodiments of the invention follow from the dependent claims.

Further, the invention aims to provide a connection to an aerosol generator, which ensures that the amount of fluid or liquid retained in the reservoir is kept substantially constant over a range of alignment or orientation angles.

The invention provides a fluid reservoir for an aerosol generator, the fluid reservoir comprising a fluid chamber for receiving a fluid or liquid, an opening for guiding the fluid or liquid received in the fluid chamber outside the fluid chamber, and a collar portion, tube portion or pipe portion surrounding the opening and extending into the fluid chamber. A first portion of the fluid chamber extends along the length of the collar portion, tube portion or pipe portion in a height direction, e.g., longitudinal direction, of the fluid chamber. A second portion of the fluid chamber is arranged adjacent to the first portion in the height direction of the fluid chamber. A lateral extension, e.g., an inner diameter, of the first portion in the directions perpendicular to the height direction of the fluid chamber is smaller than a lateral extension, e.g., an inner diameter, of the second portion in the directions perpendicular to the height direction of the fluid chamber.

The height direction of the fluid chamber may be perpendicular to a plane in which the opening lies.

The collar portion, tube portion or pipe portion surrounds the opening and extends into the fluid chamber, e.g., along the height direction of the fluid chamber.

The second portion of the fluid chamber is arranged adjacent to, e.g., above, the first portion in the height direction of the fluid chamber.

The opening may be provided in a bottom wall of the fluid chamber.

The lateral extension of the first portion in the directions perpendicular to the height direction of the fluid chamber may be smaller than the lateral extension of the remainder, i.e., the remaining portion, of the fluid chamber in the directions perpendicular to the height direction of the fluid chamber.

The length of the collar portion in the height direction, e.g., longitudinal direction, of the fluid chamber may be in the range from 2 mm to 50 mm, preferably in the range from 6 mm to 40 mm, more preferably in the range from 10 mm to 30 mm and even more preferably in the range from 12 mm to 30 mm.

If the fluid reservoir is held in an upright, i.e., vertical, position, i.e., at a holding angle of 0°, so that the height direction of the fluid chamber is oriented or aligned vertically, a predetermined portion of the fluid or liquid received in the fluid chamber is retained in a region or space of the first portion of the fluid chamber which is formed between the collar portion, i.e., an outer surface thereof, and an inner wall of the fluid chamber, i.e., of the first portion thereof. Since the portion of the fluid or liquid is retained in this region or space of the first portion of the fluid chamber, it cannot flow through the opening towards the outside of the fluid chamber, e.g., to the aerosol generator.

If the fluid reservoir is tilted or inclined relative to the upright direction, at least a part of the fluid or liquid retained in the region of the first portion of the fluid chamber formed between the collar portion and the inner wall of the fluid chamber flows to the second portion of the fluid chamber, which is arranged adjacent to the first portion in the height direction of the fluid chamber, i.e., to the part of the second portion arranged on the side of the reservoir towards which the reservoir is tilted or inclined.

Since the lateral extension of the second portion in the directions perpendicular to the height direction of the fluid chamber is larger than that of the first portion, the at least a part of the fluid or liquid retained in the region between collar portion and inner wall of the first portion of the fluid chamber is received by the second portion and thus prevented from flowing through the opening outside the fluid chamber. Hence, even when the fluid reservoir is tilted or inclined, the portion of the fluid or liquid retained initially, i.e., relative to or in the upright position of the fluid reservoir for an aerosol therapy, in the region between collar portion and inner wall of the first portion remains in the fluid chamber.

In particular, since the lateral extension of the first portion in the directions, i.e., all the directions, perpendicular to the height direction of the fluid chamber is smaller than the lateral extension of the second portion in the directions, i.e., all the directions, perpendicular to the height direction of the fluid chamber, this fluid or liquid portion is reliably retained in the fluid chamber for all tilting or inclination directions. Therefore, the retention amount of the fluid or liquid in the fluid chamber does not vary if the fluid reservoir is tilted or inclined, thus ensuring a constant retention amount over a range of alignment or orientation angles of the reservoir, e.g., within 0 to 30°, preferably within 0 to 45°, more preferably within 0 to 60°, and even more preferably within 0 to 90° in each tilting or inclination direction.

In an embodiment, the retention amount of the fluid or liquid in the fluid chamber varies, e.g., by +/-25%, preferably by +/-20%, more preferably by +/-15% and even more preferably by +/-10%, if the fluid reservoir is tilted or inclined, thus ensuring a substantially or nearly constant retention amount over a range of alignment or orientation angles of the reservoir, e.g., within 0 to 30°, preferably within 0 to 45°, more preferably within 0 to 60°, and even more preferably within 0 to 90° in each tilting or inclination direction.

The region or space of the first portion of the fluid chamber which is formed between the collar portion, i.e., an outer surface thereof, and an inner wall of the fluid chamber, i.e., of the first portion thereof, may have a volume in the range from 1 ml to 12 ml, preferably in the range from 1.5 ml to 10 ml, more preferably in the range from 2 ml to 8 ml and even more preferably in the range from 3 ml to 6 ml. In an embodiment, the volume may be in the range from 0.1 ml to 5.0 ml, preferably in the range from 0.5 ml to 4.0 ml, more preferably in the range from 0.7 ml to 3.0 ml, and even more preferably in the range from 1.0 ml to 2.0 ml.

The collar portion and the first and second portions of the fluid chamber can be manufactured in a simple and cost-efficient manner, e.g., by moulding, such as injection moulding. Further, an intentional manipulation of the retention amount of the liquid or fluid by a user or patient, e.g., by introducing objects, such as glass beads or the like, into the fluid chamber, is rendered difficult.

The region of the first portion of the fluid chamber formed between the collar portion and the inner wall of the fluid chamber may extend in an annular or ring shape, in an elliptic or oval shape, or in a polygonal shape, such as a triangular, rectangular or square shape, in the cross-section profile, i.e., in the cross-section perpendicular to the height direction of the fluid chamber.

At the transition between the first portion of the fluid chamber and the second portion of the fluid chamber, the lateral extension of the fluid chamber in the directions perpendicular to the height direction of the fluid chamber may change continuously or abruptly.

A step portion or flange portion may be formed at the transition between the first portion of the fluid chamber and the second portion of the fluid chamber. At the step portion or flange portion, there may be an abrupt change in the lateral extension, e.g., inner diameter, of the fluid chamber in the directions perpendicular to the height direction of the fluid chamber. At the step portion or flange portion, a slope or inclination of the inner wall of the fluid chamber may change abruptly.

By forming a step portion or flange portion at the transition between the first portion of the fluid chamber and the second portion of the fluid chamber, the retention amount of fluid or liquid in the fluid reservoir can be kept constant over a range of alignment or orientation angles of the reservoir in a particularly reliable manner.

The upper surface of the step portion or flange portion in the height direction of the fluid chamber may lie in a plane which is substantially perpendicular to the height direction of the fluid chamber. This arrangement enables, in a simple manner, a particularly precise control of the retention amount of fluid or liquid in the fluid chamber for a range of inclination or tilting angles of the fluid reservoir.

The upper surface of the step portion or flange portion in the height direction of the fluid chamber may lie in a plane which is substantially parallel to a plane in which the opening lies.

The upper surface of the collar portion in the height direction of the fluid chamber may lie in a plane which is substantially perpendicular to the height direction of the fluid chamber.

In an embodiment of the invention, the upper surface of the collar portion in the height direction of the fluid chamber may lie in a horizontal plane during the use of the fluid reservoir. This may include a use of the aerosol generator comprising the fluid reservoir by a user. During the aerosol generation, the upper surface of the collar portion in the height direction of the fluid chamber may lie in a horizontal plane during the use of the fluid reservoir in an aerosol generator.

The collar portion may have a substantially cylindrical shape, e.g., with a circular, elliptic or polygonal, such as triangular, rectangular or square, cross-section, e.g., in a plane perpendicular to the height direction of the fluid chamber. The collar portion may be rotationally symmetrically constructed or may be configured so as not to be rotationally symmetrical, e.g., configured so as to be rotationally symmetrical or so as not to be rotationally symmetrical with respect to the height direction of the fluid chamber.

The lateral extension of the first portion and/or the second portion of the fluid chamber in the directions perpendicular to the height direction of the fluid chamber may vary along the height direction of the fluid chamber.

The first portion of the fluid chamber may have a lateral extension, e.g., an inner diameter, in the directions perpendicular to the height direction of the fluid chamber which is substantially constant along the height direction of the fluid chamber.

The second portion of the fluid chamber may have a lateral extension, e.g., an inner diameter, in the directions perpendicular to the height direction of the fluid chamber which is substantially constant along the height direction of the fluid chamber.

The first portion of the fluid chamber and/or the second portion of the fluid chamber may have a substantially cylindrical shape, e.g., with a circular, elliptic or polygonal, such as triangular, rectangular or square, cross-section in a plane perpendicular to the height direction of the fluid chamber. The first portion of the fluid chamber and/or the second portion of the fluid chamber may be rotationally symmetrically constructed or may be configured so as not to be rotationally symmetrical, e.g., configured so as to be rotationally symmetrical or so as not to be rotationally symmetrical with respect to the height direction of the fluid chamber.

The fluid reservoir may be made of plastic, ceramic or metal. Preferably, the fluid reservoir is made of plastic. In this way, the fluid reservoir can be manufactured in a particularly simple and cost-efficient manner, e.g., by moulding, such as injection moulding.

The fluid reservoir may further comprise a filling port or filling opening for filling the fluid or liquid into the fluid chamber. In this way, the fluid chamber can be filled with the fluid or liquid in a particularly simple and precise manner.

The filling port or filling opening may be arranged above the opening for guiding the fluid or liquid received in the fluid chamber outside the fluid chamber in the height direction of the fluid chamber.

The fluid reservoir may comprise a lid or cap which closes the fluid reservoir and releasably or permanently seals the fluid chamber.

The filling port or opening may have a lid or cap to close the fluid reservoir.

The lid or cap may have a sealing line or sealing area to releasably or permanently seal the fluid reservoir, e.g., the fluid chamber, to avoid a fluid leakage from the fluid reservoir.

The lid or cap may have, e.g., for safety and practicability reasons, a guard band or string that secures the lid or cap on the fluid reservoir, the body of the aerosol generator, the aerosol generator and/or the aerosol delivery device.

The lid or cap may be releasably or inseparably connected with, for example, the fluid reservoir or the body of the aerosol generator. The connection may be realised, for example, with a ring piece around and/or in a collar area of the fluid reservoir or a collar body of the aerosol generator. The ring piece may be closely linked to the collar of the fluid reservoir or body of the aerosol generator and may be locked mechanically. For example, the ring piece may be locked mechanically into a narrowed channel on the collar area of the fluid reservoir or body of the aerosol generator. The lid or cap may be releasably or inseparably connected with, for example, the fluid reservoir or the body of the aerosol generator, e.g., with a guard band.

The one, two and/or three pieces of the lid or cap connection to the fluid reservoir or the body of the aerosol generator may be produced during a plastic injection moulding process. The lid or cap and/or the fluid reservoir and/or the aerosol generator and/or the guard band may be produced separately or combined during a plastic injection moulding process.

The lid or cap may be releasably or inseparably connected with, for example, the fluid reservoir or the body of the aerosol generator with a guard band, clamping band, holding band, retaining strap, securing strap, support strap or retaining band or the like. Therefore, the lid or cap may have a band, string, line, or cord in the material for example of the lid or cap and/or the fluid reservoir and establish an inseparable connection with the fluid reservoir or the body of the aerosol generator. The releasable or inseparable connection may be produced during a plastic injection moulding process.

The band, strap, string, line, cord etc. may have a predetermined breaking point or line. In this way, it can be reliably prevented that, after closing the fluid reservoir with the lid or cap, the lid or cap is loosened and the fluid reservoir is opened by pulling or otherwise moving the band, strap, string, line, cord etc. Therefore, an unintentional opening of the permanently sealed fluid reservoir can be avoided.

The fluid reservoir may further comprise a cover member or sealing member which releasably or permanently seals and/or covers the region or space of the first portion of the fluid chamber which is formed between the collar portion and the inner wall of the fluid chamber. By using such a cover member or sealing member, it can be reliably ensured that the portion of the fluid or liquid received in the region of the first portion formed between the collar portion and the inner wall of the fluid chamber is retained in the fluid chamber for any tilting or inclination angle of the fluid reservoir.

In this case, the fluid chamber may be filled with a predetermined amount of fluid or liquid, a portion of which is received in the region of the first portion of the fluid chamber formed between the collar portion and the inner wall of the fluid chamber. Subsequently, this region of the first portion of the fluid chamber may be sealed or covered by the cover member or sealing member, so that only the remaining portion of fluid or liquid, which is received in the fluid chamber outside this region of the first portion, is guided through the opening outside the fluid chamber, e.g., to the aerosol generator.

The cover member or sealing member may releasably or permanently seal and/or cover the region of the first portion of the fluid chamber which is formed between the collar portion and the inner wall of the fluid chamber and, simultaneously, releasably or permanently seal and/or cover the filling port or filling opening for filling the fluid or liquid into the fluid chamber. In this way, a single element, i.e., the cover member or sealing member, can be used for covering or sealing both the filling port and the region formed between the collar portion and the inner wall of the fluid chamber, thereby reducing the number of elements of the fluid reservoir and providing a simple and cost-efficient configuration.

The cover member or sealing member may be formed or arranged integrally with the lid or cap of the fluid reservoir to seal both the filling port and the region formed between the collar portion and the inner wall of the fluid chamber.

The cover member may be made of plastic, ceramic or metal. Preferably, the cover member is made of plastic. In this way, the cover member can be manufactured in a particularly simple and cost-efficient manner, e.g., by moulding, such as injection moulding.

Moreover, the present invention provides a fluid reservoir for an aerosol generator, the fluid reservoir comprising a fluid chamber for receiving a fluid or liquid, an opening for guiding the fluid or liquid received in the fluid chamber outside the fluid chamber, a recess arranged at least partly below or underneath the opening in the height direction of the fluid chamber, and a cover member or sealing member which releasably or permanently seals and/or covers the recess.

The recess may be arranged entirely below or underneath the opening in the height direction of the fluid chamber.

The opening may be provided in a bottom wall or a side wall of the fluid chamber.

The fluid reservoir may be made of plastic, ceramic or metal. Preferably, the fluid reservoir is made of plastic. In this way, the fluid reservoir can be manufactured in a particularly simple and cost-efficient manner, e.g., by moulding, such as injection moulding.

Since the cover member or sealing member releasably or permanently seals and/or covers the recess, a portion of the fluid or liquid in the fluid chamber which is received in the recess can be reliably retained in the fluid chamber for any tilting or inclination angle of the fluid reservoir, thereby allowing for the retention amount of fluid or liquid in the fluid reservoir to be kept constant for any alignment or orientation angle of the reservoir in each tilting or inclination direction.

Specifically, a fluid or liquid may be filled into the fluid chamber, wherein a portion of the fluid or liquid is received in the recess. Subsequently, the recess may be releasably or permanently sealed and/or covered by the cover member or sealing member, so that only the fluid or liquid portion arranged in the fluid chamber outside the recess is guided through the opening outside the fluid chamber, e.g., to the aerosol generator.

The fluid reservoir may further comprise a filling port or filling opening for filling the fluid or liquid into the fluid chamber. The filling port or filling opening may be arranged above the opening for guiding the fluid received in the fluid chamber outside the fluid chamber in the height direction of the fluid chamber.

The cover member or sealing member may releasably or permanently seal and/or cover the recess and, simultaneously, releasably or permanently seal and/or cover the filling port or filling opening. In this way, a single element, namely the cover member or sealing member, may be used to releasably or permanently seal and/or cover the recess and the filling port, thereby reducing the number of elements of the fluid reservoir and providing a simple and cost-efficient configuration.

The fluid reservoirs according to the present invention may be any type of fluid container, such as an ampoule, a vial or the like. The fluid container may have an opening, e.g., one opening, and/or a predetermined breaking point or line, for example, in the direction to the aerosol generator. The opening and/or predetermined breaking point or line may be positioned on the bottom area of the fluid container, such as an ampoule, a vial or the like. The fluid container, such as an ampoule, a vial or the like, may be opened via an opening element or opening mechanism. The opening element may be a thorn, pipe, cannula, needle or the like. Such an opening element is disclosed, for example, in WO-A-2007/020073, the content of which is herewith incorporated herein in its entirety by reference. Such a fluid container is disclosed, for example, in EP-A-2 062 608, the content of which is herewith incorporated herein in its entirety by reference.

The fluid reservoirs according to the present invention may be used for, e.g., in combination with, any aerosol generator, particularly a vibrating membrane aerosol generator, such as a vibrating membrane nebuliser, e.g., an electronic vibrating membrane nebuliser, an atomiser or the like. The aerosol generator may be an electronic nebuliser, e.g., a piezoelectrically driven nebuliser, i.e., a nebuliser driven by a piezoelectric element. In this case, the piezoelectric element may be arranged for vibrating or oscillating a vibratable membrane of the aerosol generator. In particular, the aerosol generator may be the aerosol generator described below.

The fluid reservoirs according to the present invention may be used, e.g., for an equivalent inhalation therapy with established drug vials which have an existing regulatory approval with predicate aerosol generators and systems, or can be used in aerosol generators adaptable to both a ventilator system or so-called tubing circuit as well as a handheld device such that an equivalent drug amount can be delivered to the user or patient by matching the different aerosol generation efficiencies of the aerosol generator during assisted breathing when connected to a ventilator versus inhaling spontaneously from a handheld device using the same drug dose and volume as approved by the FDA, EMA or other relevant regulatory authority.

The present invention provides a set of fluid reservoirs which may be used, for example, for such an equivalent inhalation therapy.

The set of fluid reservoirs comprises at least two fluid reservoirs, wherein each of the fluid reservoirs comprises a fluid chamber for receiving a fluid and an opening for guiding the fluid received in the fluid chamber outside the fluid chamber.

A first one of the at least two fluid reservoirs comprises a collar portion surrounding the opening and extending into the fluid chamber, while a second one of the at least two fluid reservoirs does not comprise such a collar portion. Otherwise, the at least two fluid reservoirs are identical or substantially identical. The first fluid reservoir may be one of the fluid reservoirs of the invention detailed above.

When using the set of fluid reservoirs, e.g., for an equivalent inhalation therapy, the first fluid reservoir is used with the handheld device system, while the second fluid reservoir is used with the ventilator system or tube system.

Due to the large flow rates in ventilator systems or tube systems, the amount of aerosol which is deposited on the walls of the tubes and connection pieces of these systems is considerably larger than the amount of aerosol which is deposited on the walls of handheld devices, resulting in an undesired variation in aerosol dosage for these two types of systems for a given amount of fluid or liquid in the fluid chamber.

This variation can be compensated by using the set of fluid reservoirs according to the present invention. Specifically, by using the first fluid reservoir, which comprises the collar portion surrounding the opening and extending into the fluid chamber, for the handheld device, the aerosol generation efficiency of this device is reduced. Since the second fluid reservoir, which does not comprise such a collar portion, is used for the ventilator system, no such efficiency reduction occurs in this system.

In this way, the amount of aerosol which is delivered to a user or patient for a given amount of fluid or liquid in the fluid chamber can be made the same or substantially the same for the handheld device and the ventilator system, thus ensuring a consistent and precise aerosol dosage for these two different types of aerosol delivery devices and administration modes.

The fluid or liquid to be received in the fluid chamber of the fluid reservoir may be a fluid or liquid for the generation of a pharmaceutical aerosol for the delivery of an active compound.

An active compound is a natural, biotechnology-derived or synthetic compound or mixture of compounds useful for the diagnosis, prevention, management or treatment of a disease, condition or symptom of an animal, in particular a human. Other terms which may be used as synonyms of active compounds include, for example, active ingredient, active pharmaceutical ingredient, drug substance, diagnostic material, drug, medicament and the like. The fluid could be of a liquid, solution, suspension, colloidal mixture or liposomal formulation form and can be prepared, mixed or opened before or during the application.

The active compound comprised in the fluid to be received in the fluid chamber may be a drug substance or a medicament which is useful for the prevention, management, diagnosis or treatment of any disease, symptom or condition affecting the body cavities, the abdomen, the eyes, the intestine, the stomach, the nose, the sinuses, the osteomeatal complex, the mouth, the trachea, the lungs, the bronchia, the bronchioles, the alveoli and/or the respiratory tract.

Among the active compounds which may be useful for serving one of the purposes named previously and that may be used together with the present invention, are, for example, substances selected from the group consisting of anti-inflammatory compounds, anti-infective agents, antiseptics, prostaglandins, endothelin receptor agonists, phosphodiesterase inhibitors, beta-2-sympathicomimetics, decongestants, vasoconstrictors, anticholinergics, immunoglobulins (e.g. Ig, IgG, IgA, IgM), immunomodulators, mucolytics, anti-allergic drugs, antihistaminics, mast-cell stabilizing agents, tumor growth inhibitory agents, wound healing agents, local anaesthetics, antioxidants, oligonucleotides, peptides, proteins, vaccines, vitamins, plant extracts, cholinesterase inhibitors, vasoactive intestinal peptide, serotonin receptor antagonists, and heparins, glucocorticoids, anti-allergic drugs, antioxidants, vitamins, leucotriene antagonists, anti-infective agents, antibiotics, antifungals, antivirals, mucolytics, decongestants, antiseptics, cytostatics, immunomodulators, vaccines, wound healing agents, local anaesthetics, oligonucleotides, xanthin derived agents, peptides, proteins and plant extracts. Such compound may be used in the form of a suspension, a solution, a colloidal formulation (i.e., liposomal), etc.

Examples of potentially useful anti-inflammatory compounds are glucocorticoids and non-steroidal anti-inflammatory agents such as betamethasone, beclomethasone, budesonide, ciclesonide, dexamethasone, desoxymethasone, fluoconolone acetonide, fluocinonide, flunisolide, fluticasone, icomethasone, rofleponide, triamcinolone acetonide, fluocortin butyl, hydrocortisone, hydroxycortisone-17-butyrate, prednicarbate, 6-methylprednisolone aceponate, mometasone furoate, dehydroepiandrosterone-sulfate (DHEAS), elastane, prostaglandin, leukotriene, bradykinin antagonists, non-steroidal anti-inflammatory drugs (NSAIDs), such as ibuprofen including any pharmaceutically acceptable salts, esters, isomers, stereoisomers, diastereomers, epimers, solvates or other hydrates, prodrugs, derivatives, or any other chemical or physical forms of active compounds comprising the respective active moieties.

Examples of anti-infective agents, whose class or therapeutic category is herein understood as comprising compounds which are effective against bacterial, fungal, and viral infections, i.e. encompassing the classes of antimicrobials, antibiotics, antifungals, antiseptics, and antivirals, are
- penicillins, including benzylpenicillins (penicillin-G-sodium, clemizone penicillin, benzathine penicillin G), phenoxypenicillins (penicillin V, propicillin), aminobenzylpenicillins (ampicillin, amoxycillin, bacampicillin), acylaminopenicillins (azlocillin, mezlocillin, piperacillin, apalcillin), carboxypenicillins (carbenicillin, ticarcillin, temocillin), isoxazolyl penicillins (oxacillin, cloxacillin, dicloxacillin, flucloxacillin), and amiidine penicillins (mecillinam);
- cephalosporins, including cefazolins (cefazolin, cefazedone); cefuroximes (cefuroxim, cefamandole, cefotiam), cefoxitins (cefoxitin, cefotetan, latamoxef, flomoxef), cefotaximes (cefotaxime, ceftriaxone, ceftizoxime, cefmenoxime), ceftazidimes (ceftazidime, cefpirome, cefepime), cefalexins (cefalexin, cefaclor, cefadroxil, cefradine, loracarbef, cefprozil), and cefiximes (cefixime, cefpodoxim proxetile, cefuroxime axetil, cefetamet pivoxil, cefotiam hexetil), loracarbef, cefepim, clavulanic acid / amoxicillin, Ceftobiprole;
- synergists, including beta-lactamase inhibitors, such as clavulanic acid, sulbactam, and tazobactam;
- carbapenems, including imipenem, cilastin, meropenem, doripenem, tebipenem, ertapenem, ritipenam, and biapenem;
- monobactams, including aztreonam;
- aminoglycosides, such as apramycin, gentamicin, amikacin, isepamicin, arbekacin, tobramycin, netilmicin, spectinomycin, streptomycin, capreomycin, neomycin, paromoycin, and kanamycin;
- macrolides, including erythromycin, clarythromycin, roxithromycin, azithromycin, dithromycin, josamycin, spiramycin and telithromycin;
- gyrase inhibitors or fluroquinolones, including ciprofloxacin, gatifloxacin, norfloxacin, ofloxacin, levofloxacin, perfloxacin, lomefloxacin, fleroxacin, garenoxacin, clinafloxacin, sitafloxacin, prulifloxacin, olamufloxacin, caderofloxacin, gemifloxacin, balofloxacin, trovafloxacin, and moxifloxacin;
- tetracyclins, including tetracyclin, oxytetracyclin, rolitetracyclin, minocyclin, doxycycline, tigecycline and aminocycline;
- glycopeptides, inlcuding vancomycin, teicoplanin, ristocetin, avoparcin, oritavancin, ramoplanin, and peptide 4;
- polypeptides, including plectasin, dalbavancin, daptomycin, oritavancin, ramoplanin, dalbavancin, telavancin, bacitracin, tyrothricin, neomycin, kanamycin, mupirocin, paromomycin, polymyxin B and colistin;
- sulfonamides, including sulfadiazine, sulfamethoxazole, sulfalene, co-trimoxazole, co-trimetrol, co-trimoxazine, and co-tetraxazine;
- azoles, including clotrimazole, oxiconazole, miconazole, ketoconazole, itraconazole, fluconazole, metronidazole, tinidazole, bifonazol, ravuconazol, posaconazol, voriconazole, and ornidazole and other antifungals including flucytosin, griseofulvin, tolnaftal, naftifin, terbinafin, amorolfin, ciclopiroxolamin, echinocandins, such as micafungin, caspofungin, anidulafungin;
- nitrofurans, including nitrofurantoin and nitrofuranzone;
- polyenes, including amphotericin B, natamycin, nystatin, flucytosine;
- other antibiotics, including tithromycin, lincomycin, clindamycin, oxazolindiones (linzezolids), ranbezolid, streptogramine A+B, pristinamycin A+B, Virginiamycin A+B, dalfopristin /quinupristin (Synercid), chloramphenicol, ethambutol, pyrazinamid, terizidon, dapson, prothionamid, fosfomycin, fucidinic acid, rifampicin, isoniazid, cycloserine, terizidone, ansamycin, lysostaphin, iclaprim, mirocin B17, clerocidin, filgrastim, formycin, and pentamidine;
- antivirals, including aciclovir, ganciclovir, birivudin, valaciclovir, zidovudine, didanosin, thiacytidin, stavudin, lamivudin, zalcitabin, ribavirin, nevirapirin, delaviridin, trifluridin, ritonavir, saquinavir, indinavir, foscarnet, amantadin, podophyllotoxin, vidarabine, tromantadine, and proteinase inhibitors, siRNA based drugs;
- antiseptics, including acridine derivatives, iodine-povidone, benzoates, rivanol, chlorhexidine, quarternary ammonium compounds, cetrimides, biphenylol, clorofene, and octenidine;
- plant extracts or ingredients, such as plant extracts from chamomile, hamamelis, echinacea, calendula, thymian, papain, pelargonium, pine trees, essential oils, myrtol, pinen, limonen, cineole, thymol, mentol, camphor, tannin, alpha-hederin, bisabolol, lycopodin, vitapherole;
- wound healing compounds including dexpantenol, allantoin, vitamins, hyaluronic acid, alpha-antitrypsin, anorganic and organic zinc salts/compounds, salts of bismuth and selen;
- interferones (alpha, beta, gamma), tumor necrosis factors, cytokines, interleukines;
- immunmodulators including methotrexat, azathioprine, cyclosporine, tacrolimus, sirolimus, rapamycin, mofetil; mofetil-mycophenolate.
- cytostatics and metastasis inhibitors;
- alkylants, such as nimustine, melphanlane, carmustine, lomustine, cyclophosphosphamide, ifosfamide, trofosfamide, chlorambucil, busulfane, treosulfane, prednimustine, thiotepa;
- antimetabolites, e.g. cytarabine, fluorouracil, methotrexate, mercaptopurine, tioguanine;
- alkaloids, such as vinblastine, vincristine, vindesine;
- antibiotics, such as alcarubicine, bleomycine, dactinomycine, daunorubicine, doxorubicine, epirubicine, idarubicine, mitomycine, plicamycine;
- complexes of transition group elements (e.g. Ti, Zr, V, Nb, Ta, Mo, W, Pt) such as carboplatinum, cis-platinum and metallocene compounds such as titanocendichloride;
- amsacrine, dacarbazine, estramustine, etoposide, beraprost, hydroxycarbamide, mitoxanthrone, procarbazine, temiposide;
- paclitaxel, gefitinib, vandetanib, erlotinib, poly-ADP-ribose-polymerase (PRAP) enzyme inhibitors, banoxantrone, gemcitabine, pemetrexed, bevacizumab, ranibizumab. Examples of potentially useful mucolytics are DNase, P2Y2-agonists (denufosol), drugs affecting chloride and sodium permeation, such as N-(3,5-Diamino-6-chloropyrazine-2-carbony)-N'-{4-[4-(2,3-dihydroxypropoxy)-phenyl]butyl}guanidine methanesulfonate (PARION 552-02), heparinoids, guaifenesin, acetylcysteine, carbocysteine, ambroxol, bromhexine, tyloxapol, lecithins, myrtol, surfactant, and recombinant surfactant proteins.

Examples of potentially useful vasoconstrictors and decongestants which may be useful to reduce the swelling of the mucosa are phenylephrine, naphazoline, tramazoline, tetryzoline, oxymetazoline, fenoxazoline, xylometazoline, epinephrine, isoprenaline, hexoprenaline, and ephedrine. Examples of potentially useful local anaesthetic agents include benzocaine, tetracaine, procaine, lidocaine and bupivacaine.

Examples of potentially useful antiallergic agents include the afore-mentioned glucocorticoids, cromolyn sodium, nedocromil, cetrizin, loratidin, montelukast, roflumilast, ziluton, omalizumab, heparinoids and other antihistamins, including azelastine, cetirizin, desloratadin, ebastin, fexofenadin, levocetirizin, loratadin.

Examples of potentially useful anticholinergic agents include ipratropium bromide, tiotropium bromide, oxitropium bromide, glycopyrrolate.

Examples of potentially useful beta-2-sympathicomimetic agents include salbutamol, fenoterol, formoterol, indacaterol, isoproterenol, metaproterenol, salmeterol, terbutaline, clenbuterol, isoetarine, pirbuterol, procaterol, ritodrine.

Examples of xanthine derived agents include theophylline, theobromine, caffeine.

Antisense oligonucleotides are short synthetic strands of DNA (or analogs) that are complimentary or antisense to a target sequence (DNA, RNA) designed to halt a biological event, such as transcription, translation or splicing. The resulting inhibition of gene expression makes oligonucleotides dependent on their composition useful for the treatment of many diseases and various compounds are currently clinically evaluated, such as ALN-RSV01 to treat the respiratory syncytical virus by, AVE-7279 to treat asthma and allergies, TPI-ASM8 to treat allergic asthma, 1018-ISS to treat cancer. Examples of potentially useful peptides and proteins include antibodies against toxins produced by microorganisms, antimicrobial peptides such as cecropins, defensins, thionins, and cathelicidins.

Moreover, the present invention provides an aerosol generator comprising the fluid reservoir according to the present invention.

The aerosol generator according to the invention provides the advantageous effects already described in detail above for the fluid reservoirs of the invention. In particular, the aerosol generator enables a high dosage accuracy of the aerosol over a range of alignment or orientation angles of the fluid reservoir and thus also the aerosol generator.

The aerosol generator may further comprise a membrane, e.g., a vibratable or oscillatable membrane, for generating an aerosol. The membrane may be arranged in a plane substantially perpendicular to the height direction of the fluid chamber. In this case, the membrane is arranged in horizontal alignment or orientation, i.e., so that the membrane lies in the horizontal plane, when the fluid reservoir is in the upright, i.e., vertical position. In this way, fluid or liquid received in the fluid chamber can be supplied to the membrane of the aerosol generator in a particularly simple manner, e.g., by gravity acting on the fluid or liquid.

The membrane may be arranged in a plane substantially parallel to the height direction of the fluid chamber. In this case, the membrane is arranged in vertical alignment or orientation, i.e., so that the membrane lies in a vertical plane, when the fluid reservoir is in the upright, i.e., vertical, position.

The aerosol generator may be a gravity-fed aerosol generator, i.e., the aerosol generator may be configured so that the fluid or liquid received in the fluid chamber is supplied from the fluid chamber through the opening to an aerosol generation portion of the aerosol generator, such as a vibratable member comprising the vibratable membrane, by gravity acting on the fluid or liquid.

The aerosol generator may be a vibrating membrane aerosol generator, such as a vibrating membrane nebuliser, e.g., an electronic vibrating membrane nebuliser, an atomiser or the like. In particular, the aerosol generator may be an electronic nebuliser, e.g., a piezoelectrically driven nebuliser, i.e., a nebuliser driven by a piezoelectric element. In this case, the piezoelectric element may be arranged for vibrating or oscillating the vibratable membrane of the aerosol generator.

The fluid reservoir may be releasably and/or permanently attached or attachable to the aerosol generator. In the former case, the fluid reservoir can be attached to and removed from the aerosol generator in a simple manner. In the latter case, the fluid reservoir can be attached to the aerosol generator and, after breaking a seal, the fluid reservoir can be removed from the aerosol generator. After breaking the seal of the fluid reservoir, the fluid reservoir may be ruined or destroyed. In this case, a reuse of the fluid reservoir is prevented.

The fluid reservoir may form an integral part of the aerosol generator.

In an embodiment, the fluid reservoir and/or the opening element and/or the collar portion may form an integral part of the aerosol generator. In this case, the opening element may be directly connected with the aerosol generator and the fluid reservoir may be releasably attached or attachable to the aerosol generator via the opening element. The opening element may be configured to open the fluid reservoir, such as an ampoule. The fluid reservoir may be sealed to the aerosol generator and, after breaking a seal, the fluid reservoir can be removed from the aerosol generator.

After breaking the seal of the fluid reservoir, the fluid reservoir may be ruined or destroyed. In this case, a reuse of the fluid reservoir is prevented. The fluid reservoir may thus be a single use element. This may have a hygienic and safety advantage, especially in the healthcare sector.

The collar portion may be part of a releasable seal of a region of the aerosol generator, e.g., the region of the first portion of the fluid chamber which is formed between the collar portion and an inner wall of the fluid chamber, and the collar portion may extend into the fluid reservoir in the height direction of the fluid chamber.

The collar portion may be configured to guide the fluid received in the fluid chamber to a membrane for generating the aerosol via gravitational force.

The present disclosure further provides an aerosol delivery device comprising the aerosol generator according to the present invention.

### Brief Description of the Drawings

Hereinafter, non-limiting examples of the present invention are explained with reference to the drawings, in which:
- Fig. 1: shows a schematic longitudinally cut cross-sectional view of a fluid reservoir according to an embodiment of the present invention;
- Fig. 2: shows a schematic perspective top view of the fluid reservoir shown in Fig. 1;
- Fig. 3: shows schematic views of a cover member according to an embodiment of the present invention, wherein Fig. 3(a) shows a schematic perspective side view of the cover member and Fig. 3(b) shows a schematic perspective bottom view of the cover member;
- Fig. 4: shows a schematic longitudinally cut cross-sectional view of the fluid reservoir shown in Fig. 1 including the cover member shown in Fig. 3;
- Fig. 5: shows schematic views of a fluid reservoir according to another embodiment of the present invention, wherein Fig. 5(a) shows a schematic longitudinally cut cross-sectional view of the fluid reservoir and Fig. 5(b) shows a schematic perspective top view of the fluid reservoir;
- Fig. 6: shows a schematic longitudinally cut cross-sectional view of the fluid reservoir shown in Fig. 5 including a cover member;
- Fig. 7: shows a schematic longitudinally cut cross-sectional view of an aerosol delivery device with an aerosol generator comprising the fluid reservoir shown in Fig. 1;
- Fig. 8: shows a schematic longitudinally cut cross-sectional view of the aerosol delivery device shown in Fig. 7 in a tilted position.
- Fig. 9: shows a schematic longitudinally cut cross-sectional view of an aerosol delivery device according to another embodiment of the present invention which may be used in a ventilator system as well as a handheld device.
- Fig. 10: shows a schematic longitudinally cut cross-sectional view of an aerosol delivery device according to yet another embodiment of the present invention which may be used in a ventilator system as well as a handheld system, e.g., with an, at least partly, removable fluid reservoir, such as a fluid or liquid ampoule.
- Fig. 11: shows a graph of experimental results for drug delivery of an aerosol delivery device that indicates the fluid amounts retained in fluid reservoirs according to embodiments of the present invention, dependent on the tilting or inclination angles of the fluid reservoirs.

### Detailed Description of Currently Preferred Embodiments

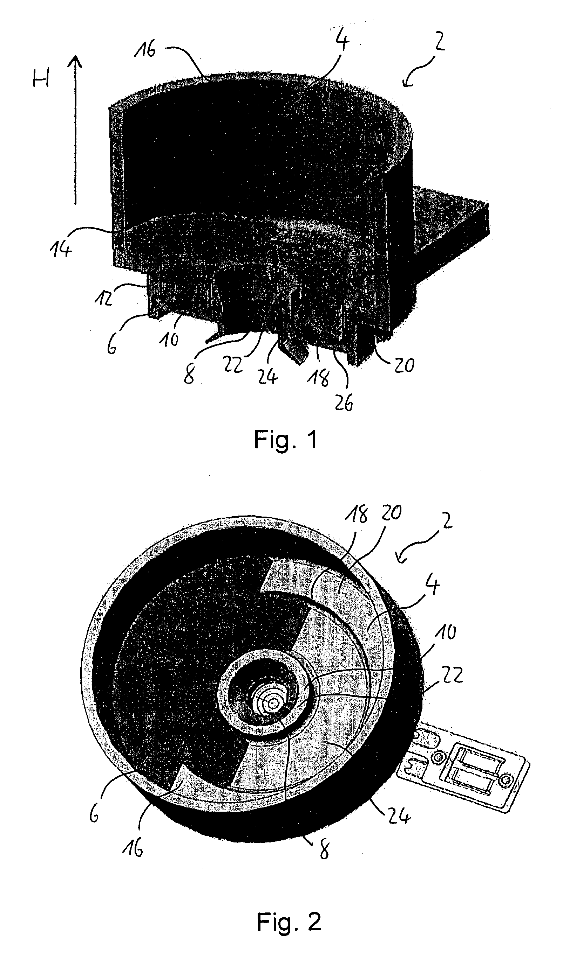

Fig. 1 shows a schematic longitudinally cut cross-sectional view of a fluid reservoir 2 according to a currently preferred embodiment of the present invention. Fig. 2 shows a schematic perspective top view of the fluid reservoir 2 shown in Fig. 1.

The fluid reservoir 2 comprises a fluid chamber 4 for receiving a fluid 6 to be aerosolised and an opening 8 for guiding the fluid 6 received in the fluid chamber 4 outside the fluid chamber 4. Further, the fluid reservoir 2 comprises a collar portion 10 surrounding the opening 8 and extending into the fluid chamber 4.

A first portion 12 of the fluid chamber 4 extends along the length of the collar portion 10 in a height direction H of the fluid chamber 4. A second portion 14 of the fluid chamber 4 is arranged adjacent to, i.e., above, the first portion 12 in the height direction H of the fluid chamber 4. A lateral extension, i.e., an inner diameter, of the first portion 12 in the directions perpendicular to the height direction H of the fluid chamber 4 is smaller than a lateral extension, i.e., an inner diameter, of the second portion 14 in the direction perpendicular to the height direction H of the fluid chamber 4.

The first and second portions 12, 14 of the fluid chamber 4 each have a cylindrical shape with a circular cross-section in a plane perpendicular to the height direction H of the fluid chamber 4. The inner diameter of the first portion 12 and the inner diameter of the second portion 14 are constant along the height direction H of the fluid chamber 4.

The collar portion 10 has a cylindrical shape with a circular cross-section in a plane perpendicular to the height direction H of the fluid chamber 4.

The opening 8 is provided in a bottom wall or in the bottom area of the side wall of the fluid chamber 4.

The fluid reservoir 2 further comprises a filling port 16 for filling the fluid 6 into the fluid chamber 4.

A step portion 18 is formed at the transition between the first portion 12 and the second portion 14. At the step portion 18, the inner diameter of the fluid chamber 4 changes abruptly, i.e., is abruptly increased from the first portion 12 to the second portion 14, as is shown from Fig. 1. The upper surface 20 of the step portion 18 in the height direction H of the fluid chamber 4 lies in a plane which is perpendicular to the height direction H of the fluid chamber 4.

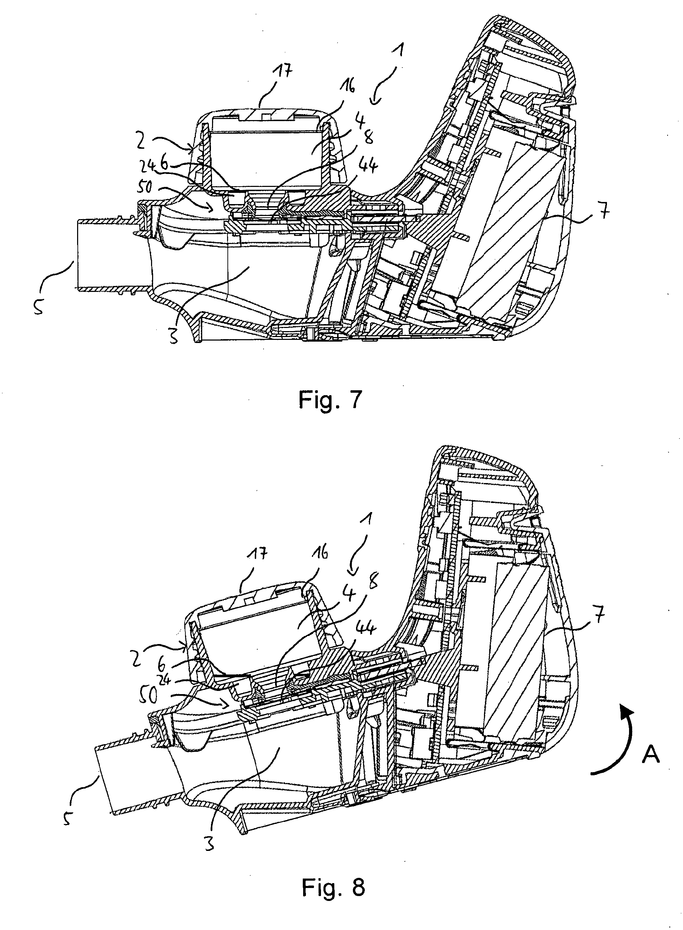

Further, the upper surface 22 of the collar portion 10 lies in a plane which is perpendicular to the height direction H of the fluid chamber 4, namely the plane in which also the upper surface 20 of the step portion 18 lies. An annular region 24 is formed between the collar portion 10, i.e., the outer surface thereof, and the inner wall 26 (not shown in Figs. 7 and Fig. 8; see Fig. 1) of the first portion 12 of the fluid chamber 4.

The fluid reservoir 2 is a retention reservoir, retaining a predetermined amount of fluid 6 therein. In the upright or vertical position of the fluid reservoir 2, in which the height direction H of the fluid chamber 4 is oriented along the vertical direction, the retained fluid 6 is received in the annular region 24. Due to the presence of the collar portion 10, the fluid 6 received in the annular region 24 cannot flow outside the fluid chamber 4 through the opening 8 and is thus reliably retained in the fluid chamber 4.

If the fluid reservoir 2 is tilted or inclined away from the upright or vertical position, as is schematically shown in Figs. 1 and 2, a portion of the fluid 6 initially retained in the annular region 24 flows over the step portion 18 and is received in the second portion 14, which has a larger inner diameter than the first portion 12. Hence, the retained fluid 6 remains in the fluid chamber 4 and does not flow through the opening 8 outside the fluid chamber 4, i.e., to an aerosol generator (described below).

Therefore, a predetermined amount of fluid 6 can be reliably retained in the fluid reservoir 2 over a range of inclination angles of the fluid reservoir 2 in each tilting or inclination direction. In this way, the amount of retained fluid 6 and thus also the aerosol dosage accuracy can be controlled in a reliable and precise manner.

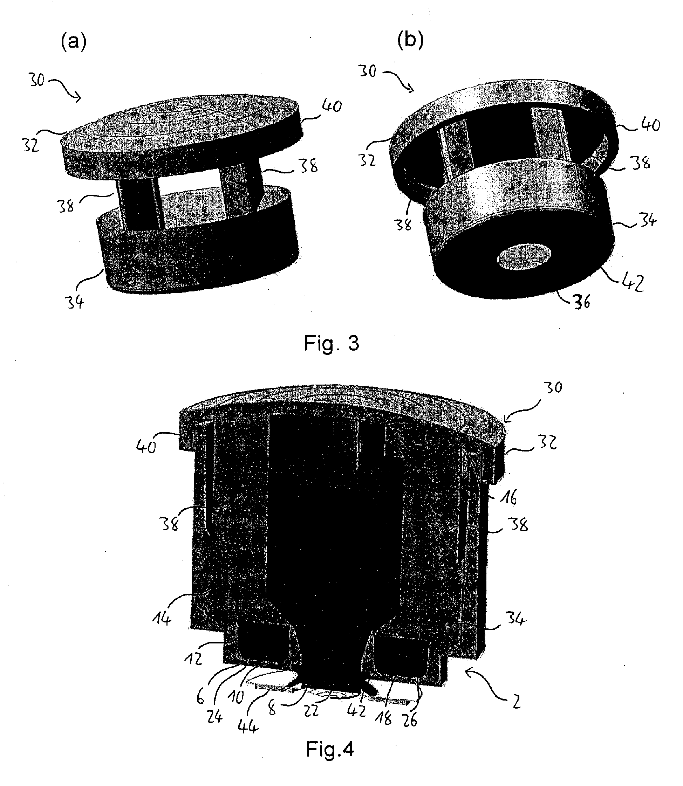

The fluid reservoir 2 may further comprise a cover member 30 which releasably or permanently seals the annular region 24 of the first portion 12 of the fluid chamber 4, as is schematically shown in Figs. 3 and 4. The cover member 30 simultaneously seals the annular region 24 and the filling port 16 (see Fig. 4).

In a further embodiment, the fluid reservoir 2 may further comprise a lid or cap 17 to close the fluid reservoir 2 in the bottom area of the fluid reservoir 2. The lid or cap 17 may releasably or permanently seal the fluid reservoir 2 in the top area of the fluid reservoir 2, to prevent a fluid or liquid leakage or loss.

The lid or cap 17 may be inseparably connected with the fluid reservoir 2 or the body of the aerosol generator with a guard band, clamping band, holding band, retaining strap, securing strap, support strap or retaining band or the like. Therefore, the lid or cap 17 may have a band, string, line, or cord in the material for example of the lid or cap 17 and/or the fluid reservoir 2 and establish an inseparable connection with the fluid reservoir 2 or the body of the aerosol generator. The inseparable connection may be produced during a plastic injection moulding process.

The cover member 30 has a substantially disc-shaped upper portion 32 and an annular lower portion 34 with an opening 36. The upper portion 32 and the lower portion 34 of the cover member 30 are connected to each other by a pair of connection members 38, such as struts, rods or bars.

The cover member 30 may comprise one or more connection members, e.g., two, three, four, five or six connection members.

The fluid reservoir 2 and the cover member 30 are made of plastic and formed by moulding, e.g., injection moulding.

The upper portion 32 of the cover member 30 has a collar 40 which fits over the wall of the upper end of the fluid reservoir 2 (Fig. 4), thus allowing for the filling port 16 to be sealed by the upper portion 32.

When the cover member 30 is attached to the remainder of the fluid reservoir 2, the upper portion 32 releasably or permanently seals the filling port 16 and the lower portion 34 releasably or permanently seals the annular region 24 of the first portion 12 of the fluid chamber 4, as is schematically shown in Fig. 4.

Specifically, the lower portion 34 has an outer diameter which is larger than the inner diameter of the first portion 12 of the fluid chamber 4, i.e., the inner diameter of the annular region 24. Further, the inner diameter of the opening 36 of the lower portion 34 is substantially the same as the inner diameter of the collar portion 10 at the upper end thereof. Hence, when the cover member 30 is attached to the remainder of the fluid reservoir 2, a lower surface 42 of the lower portion 34 rests on at least a part of the upper surface 20 of the step portion 18 and at least a part of the upper surface 22 of the collar portion 10, thus sealing the annular region 24 (Fig. 4).

Therefore, the fluid 6 received in the annular region 24 is safely retained therein, independent of the tilting or inclination angle of the fluid reservoir 2, while the fluid 6 received in the remaining portion of the fluid chamber 4 can freely flow outside the fluid chamber 4 through the opening 36 of the lower portion 34 of the cover member 30 and the opening 8 of the fluid reservoir 2. From the opening 8, the fluid 6 can flow directly to an aerosol generator (described below), e.g., to a vibrating membrane 44 thereof, as is schematically shown in Fig. 4.

Thus, a predetermined amount of fluid 6 can be retained in the fluid reservoir 2 comprising the cover member 30, entirely independent of a tilting or inclination angle of the fluid reservoir 2. In this way, the amount of retained fluid 6 and thus also the aerosol dosage accuracy can be controlled in a particularly precise and reliable manner.

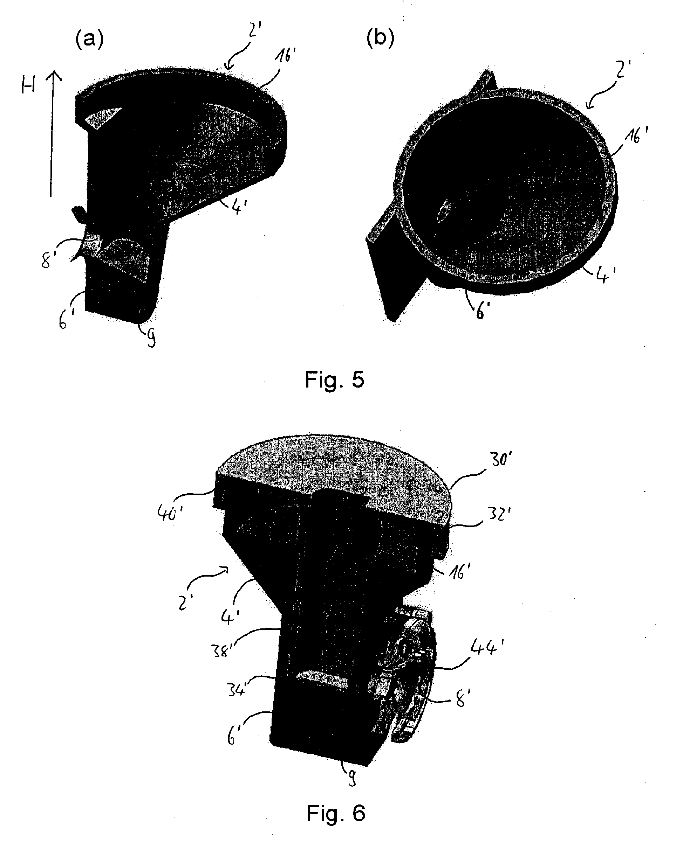

Figs. 5 and 6 show schematic views of a fluid reservoir 2' according to another currently preferred embodiment of the present invention.

The fluid reservoir 2' comprises a fluid chamber 4' for receiving a fluid 6' and an opening 8' for guiding the fluid 6' received in the fluid chamber 4' outside the fluid chamber 4'. Further, the fluid reservoir 2' comprises a recess 9 arranged below the opening 8' in the height direction H of the fluid chamber 4' and a cover member 30' which releasably or permanently seals the recess 9.

Moreover, the fluid reservoir 2' has a filling port 16' for filling the fluid 6' into the fluid chamber 4'. The opening 8' for guiding the fluid 6' received in the fluid chamber 4' outside the fluid chamber 4' is provided in a sidewall of the fluid chamber 4'.

The fluid reservoir 2' and the cover member 30' are made of plastic and formed by moulding, e.g., injection moulding.

The cover member 30' comprises a substantially disc-shaped upper portion 32' and a lower portion 34' which is connected to the upper portion 32' with a connection member 38', such as a strut, a rod or a bar. The cross-sectional shape and size of the lower portion 34' in a plane perpendicular to the height direction of the fluid chamber 4' are substantially the same as those of the upper portion of the recess 9. The upper portion 32' of the cover member 30' has a collar 40' which fits over the wall of the fluid reservoir 2' at the upper end thereof (Fig. 6).

The opening 8' is provided in a side wall of the fluid reservoir 2'. Hence, the fluid reservoir 2' according to this embodiment may be particularly advantageously used for an aerosol generator with a membrane 44' arranged in a plane substantially parallel to the height direction H of the fluid chamber 4', as is schematically shown in Fig. 6.

When the cover member 30' is attached to the remainder of the fluid reservoir 2', the upper portion 32' releasably or permanently seals the filling port 16' and the lower portion 34' releasably or permanently seals the recess 9, as is shown in Fig. 6. Hence, the fluid 6' received in the recess 9 is safely retained in the fluid chamber 4', independent of a tilting or inclination angle of the fluid reservoir 2', while the fluid 6' received in the remaining portion of the fluid chamber 4' can freely flow past the connection element 38' through the opening 8' towards the membrane 44' of the aerosol generator.

Hence, the fluid reservoir 2' allows for a predetermined amount of fluid 6' to be retained in the fluid reservoir 2', substantially or nearly independent of a feasible tilting or inclination angle of the fluid reservoir 2'.

An amount of fluid 6' retained in the fluid reservoir 2' may be substantially or nearly constant for tilting or inclination angles in the range of 0° to 45°. An amount of fluid 6' retained in the fluid reservoir 2' may be constant for tilting or inclination angles in the range of 0° to 30°. An amount of fluid 6' retained in the fluid reservoir 2' may be entirely constant for tilting or inclination angles in the range of 0° to 15°. Thus, the amount of retained fluid 6' and, therefore, also the aerosol dosage accuracy, i.e. the drug delivery of the aerosol delivery device, can be controlled in a particularly precise and reliable manner.

Experimental results for drug delivery of an aerosol delivery device indicating corresponding fluid amounts retained in fluid reservoirs according to the present invention are shown in Table 1 below for different inclination angles and directions (backward, forward, sideward) of the fluid reservoirs. The terms "No. 14/1", "No. 14/2" and "No. 14/3" in the first column of Table 1 denote three fluid reservoirs which have the same configuration, namely the configuration shown in Figs. 1 and 2 and described in detail above.

Table 1 shows values for the drug delivery (DD%) of an aerosol delivery device ex mouthpiece of the aerosol delivery device under simulated breathing conditions. The drug delivery is the ratio of the amount of aerosolised drug collected on inspiratory filters to the amount of drug filled into the fluid reservoir. In Table 1, the drug delivery is given in percent [%] for the measurements "No. 14/1", "No. 14/2" and "No. 14/3" for different inclination angles and directions. This value (DD%) is influenced by the total aerosol delivery device setup and depends on many design parameters. Specifically, variables which may influence this value may be the fluid reservoir 2, 2', 2" (and the retention amount of the fluid or liquid in the fluid chamber), as well as the characteristics of an aerosol chamber of the aerosol delivery device (with aerosol deposition during air flow or gas flow) and the functionality (hysteresis) of inlet and outlet one-way valves on the aerosol chamber. In order to minimise the effect of such possible influences on the measurements, the same setup for the aerosol delivery device was used for all the measurements shown in Table 1.

The mean value of the delivered dose, which is denoted by the term "Mean" in the first column of Table 1, was calculated for the prototype delivered dose (DD) measurements "No. 14/1", "No. 14/2" and "No. 14/3" for each inclination angle of the aerosol delivery device. The term "Reference" in the first column of Table 1 denotes the ratio of the mean value ("Mean" in Table 1) of the delivered dose (DD%) for a given inclination angle to the mean value of the delivered dose (DD%) for an inclination angle of 0° or, in other words, for the horizontal orientation of the aerosol delivery device. Thus, the Reference ratio is 100% for the "base" case (see the second column of Table 1). The ratios "Reference" in Table 1 are given in percent.

The drug solution used for the experiments shown in Table 1 was Salbutamol, specifically "Sultanol 1.25mg/2.5ml" from the manufacturer and pharmaceutical company GSK.

The aerosol characterisation and delivered dose measurements were done with the PARI Compass II breathing simulator with the software v1.0. The simulations were done corresponding to Ph. Eur. 2.9.44 (Breathing simulator specifications), with a breathing pattern for adults, a tidal volume of 500 ml, a frequency of 15 cycles/min and a sinusoidal waveform.

The compound "Sultanol" was measured via the HPLC method, including a Waters Alliance 2695 pump/autosampler, a Waters 2996 PDA detector, and a Waters Empower 3 data handling system.

**Table 1**

| | 0° base | 15° backward | 15° forward | 15° sideward | 30° backward | 45° backward | 45° forward | 45° sideward |
|---|---|---|---|---|---|---|---|---|
| No. 14/1 [DD%] | 30.4 | 33.3 | 32.9 | 28.3 | 29.5 | 22.1 | 19.1 | 30.1 |
| No. 14/2 [DD%] | 30.6 | 34 | 27.7 | 29.7 | 28.8 | 21.3 | 18.8 | 28.5 |
| No. 14/3 [DD%] | 32.4 | 29.2 | 29.4 | 29.8 | 28.2 | 19.8 | 20.6 | 27.7 |
| Mean [DD%] | 31.1 | 32.2 | 30 | 29.3 | 28.8 | 21.1 | 19.5 | 28.8 |
| Reference [%] | 100 | 104 | 96 | 94 | 93 | 68 | 63 | 93 |

Further experimental delivered dose (DD) results for the indication of the fluid amounts retained in fluid reservoirs according to the present invention are shown in Table 2 below for different inclination angles and directions (backward, forward, sideward) of the fluid reservoirs. The terms "No. 17/1", "No. 17/2" and "No. 17/3" in the first column of Table 2 denote three fluid reservoirs which have the same configuration, namely the configuration shown in Figs. 1 and 2 and described in detail above. The prototypes "No. 17/1", "No. 17/2" and "No. 17/3" are produced in different test productions than "No. 14/1", "No. 14/2" and "No. 14/3". The values for the delivered dose ("[DD%]"), the mean value of the delivered dose ("Mean") and the ratio "Reference" given in Table 2 were obtained in the same manner as described above for Table 1. In particular, the same drug solution was used.

**Table 2**

| | 0° base | 30° backward | 30° forward | 30° sideward | 45° backward | 45° sideward |
|---|---|---|---|---|---|---|
| No. 17/1 [DD%] | 30.4 | 34 | 34.4 | 32.3 | 20.7 | 20.3 |
| No. 17/2 [DD%] | 30.6 | 32.9 | 37 | 33.2 | 25.4 | 26.6 |
| No. 17/3 [DD%] | 32.4 | 32.3 | 35.2 | 31.6 | 22.9 | 23.7 |
| Mean[DD%] | 31.1 | 33.1 | 35.5 | 32.4 | 23 | 23.6 |
| Reference [%] | 100 | 106 | 114 | 104 | 74 | 76 |

As can be seen from Tables 1 and 2, the delivered dose and, thus, corresponding amount of fluid retained in the fluid reservoirs is approximately constant for inclination angles in the range of 0° to 30°. Small variations in the delivered dose and retained fluid amount were observed only in some of the inclination directions for an inclination angle of 45°.

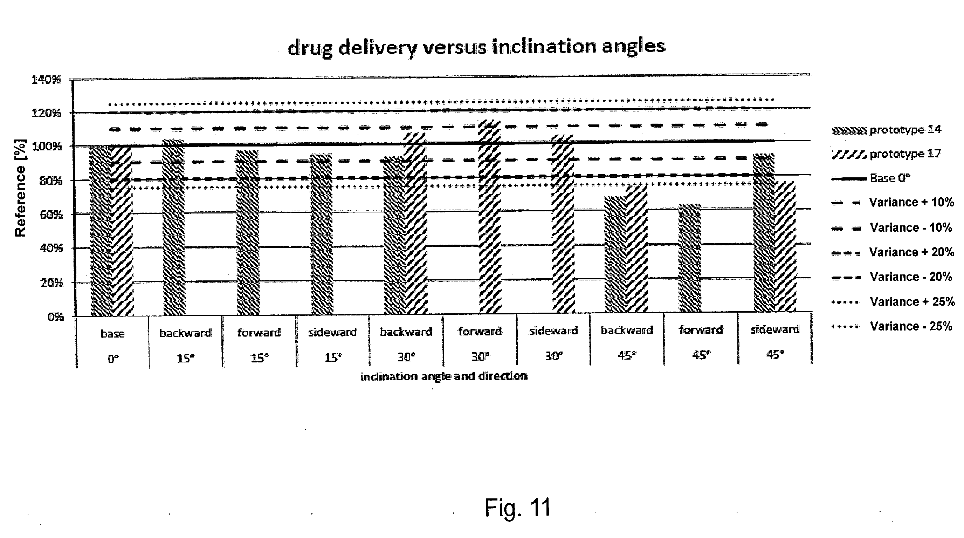

The values of the ratio "Reference" given in Tables 1 and 2 are shown in the graph of Fig. 11 for the fluid reservoirs "No. 14/1", "No. 14/2" and "No. 14/3" ("prototype 14" in Fig. 11) and the fluid reservoirs "No. 17/1", "No. 17/2" and "No. 17/3" ("prototype 17" in Fig. 11). The values of the ratio "Reference" are given on the ordinate of the graph of Fig. 11 and the inclination angles (arc degree [°]) and directions (forward, sideward, backward) are indicated on the abscissa of the graph of Fig. 11. Further, values for variations of the ratio "Reference" by +/- 25%, +/- 20% and +/- 10%, respectively, as compared to the ratio for the upright position of the fluid reservoir ("base" in Tables 1 and 2 and Fig. 11) are indicated in Fig. 11 by dashed and dotted lines.

As is shown in Fig. 11, the ratio "Reference" obtained in the "prototype 14" measurements exhibits only small variations of less than 10% for inclination angles up to 30°.

Figs. 7 and 8 show schematic cross-sectional views of an aerosol delivery device 1 with an aerosol generator 50 according to a currently preferred embodiment of the present invention, comprising the fluid reservoir 2 shown in Figs. 1 and 2. Fig. 7 shows the aerosol delivery device in an upright position, with the height direction H of the fluid chamber 4 of the fluid reservoir 2 oriented along the vertical direction, and Fig. 8 shows the aerosol delivery device 1 in a tilted or inclined position, as is indicated by arrow A.

The aerosol delivery device 1 comprises the aerosol generator 50, an aerosol chamber 3, a mouthpiece 5 and an energy source 7, such as a battery, supplying energy to the aerosol generator 50 for aerosol generation.

The aerosol generator 50 comprises the fluid reservoir 2, the membrane 44 and a vibrator (not shown), such as a piezoelectric element, for vibrating the membrane 44. The membrane 44 has a plurality of holes or openings (not shown). The fluid reservoir 2 comprises a screw cap 17 for sealing the filling port 16 of the fluid reservoir 2.

The aerosol generator 50 is a vibrating membrane aerosol generator, wherein the membrane 44 for generating an aerosol is arranged in a plane perpendicular to the height direction H of the fluid chamber 4.

In the following, operation of the aerosol delivery device 1 for the generation and delivery of an aerosol will be described.

The fluid 6 to be aerosolised, for example, a fluid comprising an active compound, such as a drug substance or a medicament, is filled into the fluid chamber 4 through the filling port 16. After filling the fluid 6 into the fluid chamber 4, the filling port 16 is sealed by attaching the screw cap 17 to the fluid reservoir 2.

The fluid 6 received in the fluid chamber 4 outside the annular region 24 is supplied through the opening 8 to the membrane 44 of the aerosol generator 50 by gravity acting on the fluid 6. Thus, the aerosol generator 50 is a gravity-fed aerosol generator.

Energy is supplied from the energy source 7 to the vibrator (not shown) of the aerosol generator 50, activating the vibrator and thus causing the membrane 44 to vibrate.

The fluid 6 supplied to the membrane 44 through the opening 8 is conveyed through the holes or openings (not shown) in the vibrating membrane 44 and thereby aerosolised into the aerosol chamber 3 of the aerosol delivery device 1. The aerosol thus provided in the aerosol chamber 3 is inhaled by a user or patient through the mouthpiece 5, which is arranged in fluid communication with the aerosol chamber 3.

The fluid 6 received in the annular region 24 between the collar portion 10 and the inner wall 26 (not shown in Fig. 7 and Fig. 8; see Fig. 1) of the fluid chamber 4 is safely retained in the fluid chamber 4, as is schematically shown in Fig. 7. If the aerosol delivery device 1 is in a tilted or inclined position (Fig. 8), a portion of the fluid retained in the fluid chamber 4 is received by the second portion 14 (not shown in Fig. 7 and Fig. 8; see Fig. 1) of the fluid chamber 4, as has been detailed above. Therefore, even if the aerosol delivery device 1 is moved, i.e., tilted or inclined, by the user or patient during aerosol therapy, it is ensured that the predetermined amount of fluid 6 is reliably retained in the fluid reservoir 2, so that the aerosol dosage accuracy can be controlled in a precise and reliable manner.

Thus, the aerosol delivery device 1 according to this embodiment can be particularly advantageously used as a handheld device which is held by the user or patient during aerosol therapy.

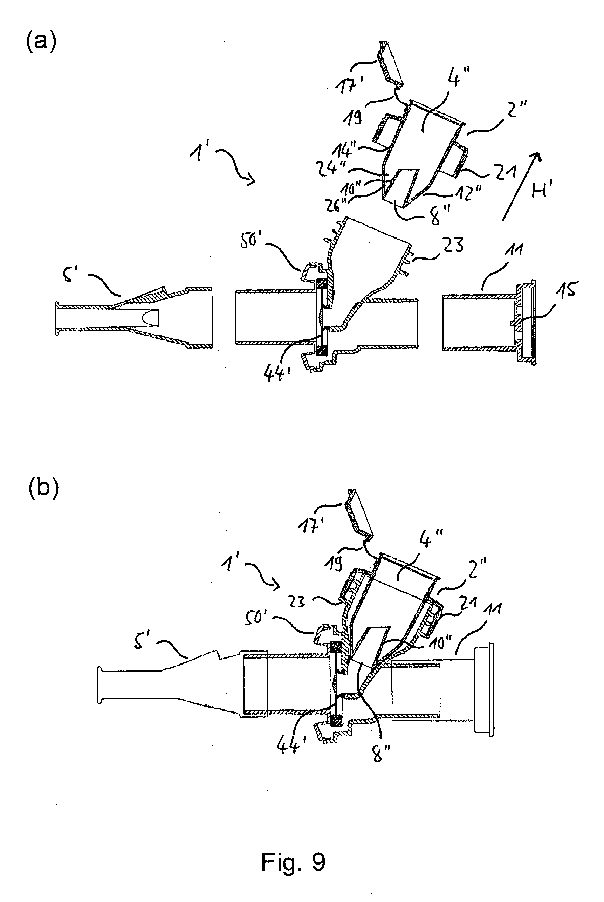

Fig. 9 shows a schematic longitudinally cut cross-sectional view of an aerosol delivery device 1' according to another embodiment of the present invention which may be used for a ventilator system (or tube system) as well as a handheld device, wherein the use as a handheld device is illustrated in the Figure. Fig. 9(a) is an exploded view of the aerosol delivery device 1' and Fig. 9(b) shows the aerosol delivery device 1' in the assembled state thereof.

The aerosol delivery device 1' comprises an aerosol generator 50', a fluid reservoir 2", a mouthpiece 5' and an endpiece 11.

The aerosol generator 50' comprises a membrane 44' and a vibrator (not shown), such as a piezoelectric element, for vibrating the membrane 44'. The membrane 44' has a plurality of holes or openings (not shown).

The aerosol generator 50' is a vibrating membrane aerosol generator. The aerosol generator 50' differs from the aerosol generator 50 mainly in that the membrane 44' is arranged in a vertical rather than a horizontal alignment.

The fluid reservoir 2" comprises a lid or cap 17' for sealing the fluid chamber 4". The lid or cap 17' is secured to the fluid reservoir 2" with a band 19, so as to establish an inseparable connection with the fluid reservoir 2".

The fluid reservoir 2" is attachable to and removable from the aerosol generator 50'. The fluid reservoir 2" has a threaded collar 21 that can be screwed to a corresponding threaded portion 23 provided on the aerosol generator 50', thus allowing for the fluid reservoir 2" to be securely mounted to the aerosol generator 50'.

As is shown in Figs. 9(a) and (b), the fluid reservoir 2" comprises a fluid chamber 4" for receiving a fluid (not shown) to be aerosolised and an opening 8" for guiding the fluid received in the fluid chamber 4" outside the fluid chamber 4". Further, the fluid reservoir 2" comprises a collar portion 10" surrounding the opening 8" and extending into the fluid chamber 4". As is illustrated in Figs. 9(a) and (b), the collar portion 10" is configured so as not to be rotationally symmetrical with respect to a height direction H' of the fluid chamber 4".

A first portion 12" of the fluid chamber 4" extends along the length of the collar portion 10" in the height direction H' of the fluid chamber 4". A second portion 14" of the fluid chamber 4" is arranged adjacent to, i.e., above, the first portion 12" in the height direction H' of the fluid chamber 4". A lateral extension of the first portion 12" in the directions perpendicular to the height direction H' of the fluid chamber 4" is smaller than a lateral extension of the second portion 14" in the directions perpendicular to the height direction H' of the fluid chamber 4".

The lateral extension of the first portion 12" of the fluid chamber 4" in the directions perpendicular to the height direction H' of the fluid chamber 4" varies along the height direction H' of the fluid chamber 4", as is shown in Figs. 9(a) and (b).

A region 24" is formed between the collar portion 10", i.e., the outer surface thereof, and an inner wall 26" of the first portion 12" of the fluid chamber 4".

In the fully assembled state of the aerosol delivery device 1', the fluid reservoir 2" is positioned relative to the aerosol generator 50' in such a way that the height direction H' of the fluid chamber 4" is arranged at an angle, i.e., tilted, with respect to the plane of the membrane 44', as is shown in Fig. 9(b).

The operation of the aerosol delivery device 1' for the generation and delivery of an aerosol is similar to that of the aerosol delivery device 1 described above.

The fluid (not shown) to be aerosolised, for example, a fluid comprising an active compound, such as a drug substance or a medicament, is filled into the fluid chamber 4" after attachment of the fluid reservoir 2" to the aerosol generator 50' (see Fig. 9(b)). After filling the fluid into the fluid chamber 4", the fluid chamber 4" is sealed by the lid or cap 17'.

The fluid received in the fluid chamber 4" outside the region 24" is supplied through the opening 8" to the membrane 44' of the aerosol generator 50' by gravity acting on the fluid. Thus, the aerosol generator 50' is a gravity-fed aerosol generator.

The vibrator (not shown) of the aerosol generator 50' is activated, thus causing the membrane 44' to vibrate.

The fluid supplied to the membrane 44' through the opening 8" is conveyed through the holes or openings (not shown) in the vibrating membrane 44' and thereby aerosolised. The aerosol thus provided is inhaled by a user or patient through the mouthpiece 5', which is arranged in fluid communication with the membrane 44'. A valve 15 provided in the endpiece 11 (see Fig. 9(a)) allows for the flow of air, e.g., ambient air, into the aerosol generator 50'.

The fluid received in the region 24" between the collar portion 10" and the inner wall 26" of the fluid chamber 4" is safely retained in the fluid chamber 4".

The fluid reservoir 2" may be particularly advantageously used as part of a set of fluid reservoirs which may be employed, for example, for an equivalent inhalation therapy, as has been described above.

This set of fluid reservoirs may comprise at least two fluid reservoirs, wherein the fluid reservoir 2" may be a first one of the at least two fluid reservoirs. A second one of the at least two fluid reservoirs may be a fluid reservoir which is substantially identical to the fluid reservoir 2" but does not comprise a collar portion. This second fluid reservoir is thus obtained by removing the collar portion 10" from the fluid reservoir 2".

As has been detailed above, the fluid reservoir 2" can be advantageously used for a handheld device, such as the aerosol delivery device 1'.

The second fluid reservoir, which is obtained by removing the collar portion 10" from the fluid reservoir 2", may be used for a ventilator system.

In this way, variations in aerosol dosage for these two types of systems can be compensated, as has been explained in detail above. Specifically, by using the first fluid reservoir 2" for the handheld device, the aerosol generation efficiency of this device is reduced. Since the second fluid reservoir is used for the ventilator system, no such efficiency reduction occurs in this system.

In this way, the amount of aerosol which is delivered to a user or patient for a given amount of fluid or liquid in the fluid chamber can be made the same or substantially the same for the handheld device and the ventilator system, thus ensuring a consistent and precise aerosol dosage for these two different types of aerosol delivery devices.

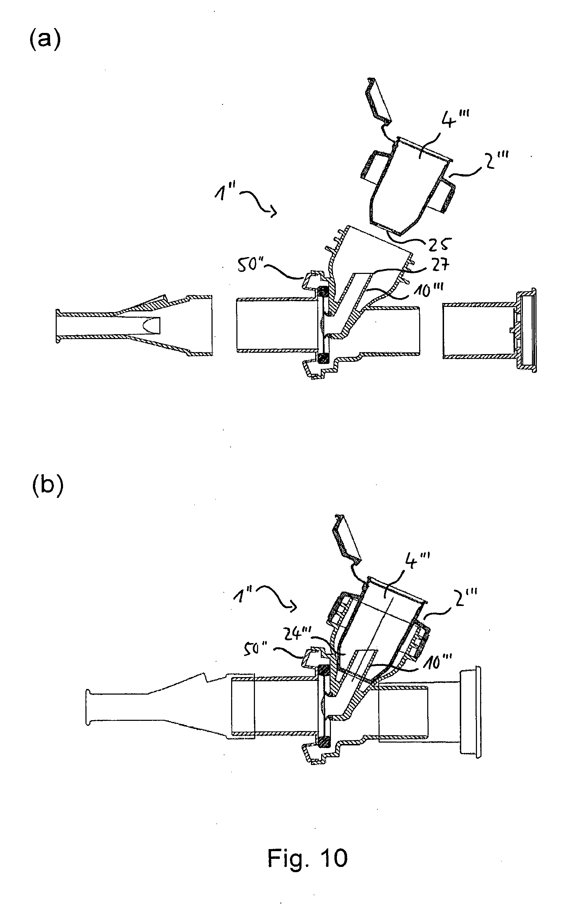

Fig. 10 shows a schematic longitudinally cut cross-sectional view of an aerosol delivery device 1" according to yet another embodiment of the present invention which may be used in a ventilator system as well as a handheld system, e.g., with an, at least partly, removable fluid reservoir, such as a fluid or liquid ampoule, wherein the use as a handheld device is illustrated in the Figure. Fig. 10(a) is an exploded view of the aerosol delivery device 1" and Fig. 10(b) shows the aerosol delivery device 1" in the assembled state thereof.

The aerosol delivery device 1" differs from the aerosol delivery device 1' in that the collar portion 10"' forms part of the aerosol generator 50". Further, the fluid reservoir 2"' has a closed bottom portion 25, which may have a predetermined breaking point or line (not shown). The collar portion 10"' is provided with a cutting means 27, such as a sharp edge or the like, at an upper portion thereof.

The remaining parts of the aerosol delivery device 1" are identical to those of the aerosol delivery device 1' and a repeated description thereof is thus omitted.

When attaching the fluid reservoir 2"' to the aerosol generator 50", the cutting means 27 of the collar portion 10'" pierces the bottom portion 25 of the fluid reservoir 2"', thus creating an opening therein. Subsequently, upon screwing the fluid reservoir 2"' further to the aerosol generator 50", the collar portion 10"' is introduced into the fluid chamber 4"', so as to surround the opening created in the bottom portion 25 and extending into the fluid chamber 4"' (see Fig. 10(b)).

In this way, in the assembled state of the aerosol delivery device 1", a region 24"' is formed in the fluid chamber 4"', which is substantially the same as the region 24" of the fluid reservoir 2". Hence, the aerosol delivery device 1" can be used substantially in the same manner as the aerosol delivery device 1' described above.

In particular, in substantially the same way as the fluid reservoir 2", the fluid reservoir 2"' can be used as part of a set of fluid reservoirs which may be employed, for example, for an equivalent inhalation therapy, as has been described above. In this case, for use as a handheld device, an aerosol generator with a collar portion is employed, and, in the second use case with a ventilator system, an aerosol generator without a collar portion or a reduced collar portion is employed. This aerosol generator can be obtained by removing the collar portion 10"' from the aerosol generator 50" or reducing the collar portion 10"' in the height direction H', so that substantially the whole fluid from the fluid reservoir 2"' can reach the aerosol generator.

The operation of the aerosol delivery device 1" is substantially the same as that of the aerosol delivery device 1' and a repeated description thereof is thus omitted. However, due to the presence of the initially closed bottom portion 25, the aerosol delivery device 1" offers the additional possibility of filling a fluid into the fluid chamber 4"' prior to attaching the fluid reservoir 2"' to the aerosol generator 50".

The foregoing embodiments and their variants have been disclosed for illustrative purposes only, and further variation is wholly possible within the capabilities of the skilled reader. Accordingly, the appended claims are intended to cover all modifications, substitutions, alterations, omissions and additions which one skilled in the art could achieve from the foregoing disclosure, taking into account his own general and specialist knowledge and expertise.

## Claims

1. A fluid reservoir (2) for an aerosol generator (50), the fluid reservoir (2) comprising
- a fluid chamber (4) for receiving a fluid (6),
- an opening (8) for guiding the fluid (6) received in the fluid chamber (4) outside the fluid chamber (4), and
- a collar portion (10) surrounding the opening (8) and extending into the fluid chamber (4),
wherein
- a first portion (12) of the fluid chamber (4) extends along the length of the collar portion (10) in a height direction (H) of the fluid chamber (4),
- a second portion (14) of the fluid chamber (4) is arranged adjacent to the first portion (12) in the height direction (H) of the fluid chamber (4), and
- a lateral extension of the first portion (12) in the directions perpendicular to the height direction (H) of the fluid chamber (4) is smaller than a lateral extension of the second portion (14) in the directions perpendicular to the height direction (H) of the fluid chamber (4).

2. The fluid reservoir (2) according to claim 1, wherein a step portion (18) is formed at the transition between the first portion (12) of the fluid chamber (4) and the second portion (14) of the fluid chamber (4).

3. The fluid reservoir (2) according to claim 2, wherein the upper surface (20) of the step portion (18) in the height direction (H) of the fluid chamber (4) lies in a plane which is substantially perpendicular to the height direction (H) of the fluid chamber (4).

4. The fluid reservoir (2) according to any one of the preceding claims, wherein the first portion (12) of the fluid chamber (4) has an inner diameter which is substantially constant along the height direction (H) of the fluid chamber (4).

5. The fluid reservoir (2) according to any one of the preceding claims, wherein the second portion (14) of the fluid chamber (4) has an inner diameter which is substantially constant along the height direction (H) of the fluid chamber (4).

6. The fluid reservoir (2) according to any one of the preceding claims, wherein the first portion (12) of the fluid chamber (4) and/or the second portion (14) of the fluid chamber (4) has a rotationally symmetrical shape.

7. The fluid reservoir (2) according to any one of the preceding claims, wherein the first portion (12) of the fluid chamber (4) and/or the second portion (14) of the fluid chamber (4) has a substantially cylindrical shape.

8. The fluid reservoir (2) according to any one of the preceding claims, further comprising a filling port (16) for filling the fluid (6) into the fluid chamber (4).

9. The fluid reservoir (2) according to any one of the preceding claims, further comprising a lid or cap (17) which closes the fluid reservoir (2) and releasably or permanently seals the fluid chamber (4).

10. The fluid reservoir (2) according to any one of the preceding claims, further comprising a cover member (30) which releasably or permanently seals a region (24) of the first portion (12) of the fluid chamber (4) which is formed between the collar portion (10) and an inner wall (26) of the fluid chamber (4).

11. The fluid reservoir (2) according to claim 10 as dependent on claim 8, wherein the cover member (30) releasably or permanently seals the region (24) of the first portion (12) of the fluid chamber (4) which is formed between the collar portion (10) and the inner wall (26) of the fluid chamber (4) and releasably or permanently seals the filling port (16).

12. A fluid reservoir (2') for an aerosol generator (50), the fluid reservoir (2') comprising
- a fluid chamber (4') for receiving a fluid (6'),
- an opening (8') for guiding the fluid (6') received in the fluid chamber (4') outside the fluid chamber (4').
- a recess (9) arranged at least partly below the opening (8') in a height direction (H) of the fluid chamber (4'), and
- a cover member (30') which releasably or permanently seals the recess (9).

13. The fluid reservoir (2') according to claim 12, further comprising a filling port (16') for filling the fluid (6') into the fluid chamber (4'), wherein the cover member (30') releasably or permanently seals the recess (9) and the filling port (16').

14. An aerosol generator (50), comprising the fluid reservoir (2, 2') according to any one of the preceding claims.

15. The aerosol generator (50) according to claim 14, further comprising a membrane (44) for generating an aerosol, wherein the membrane (44) is arranged in a plane perpendicular to the height direction (H) of the fluid chamber (4).

16. The aerosol generator (50) according to claim 14 or 15, wherein the aerosol generator (50) is configured so that the fluid (6) is supplied from the fluid chamber (4) through the opening (8) to an aerosol generator by gravity.

17. The aerosol generator (50) according to any one of claims 14 to 16, wherein the aerosol generator (50) is a vibrating membrane aerosol generator.

18. The aerosol generator (50) according to any one of claims 14 to 17, wherein the collar portion (10) is part of a releasable seal of a region (24) of the aerosol generator (50) and the collar portion (10) extends into the fluid reservoir (2) in the height direction (H) of the fluid chamber (4').

19. The aerosol generator (50) according to any one of claims 14 to 18, wherein the collar portion (10) is configured to guide the fluid (6) received in the fluid chamber (4) to a membrane for generating the aerosol via gravitational force.
